# FASCICULE DE BREVET EUROPEEN

(11) **EP 1 966 260 B1**
(45) Date de publication et mention de la délivrance du brevet: **06.06.2012**
(21) Numéro de dépôt: 06842165.0
(22) Date de dépôt: 14.12.2006
(51) Int. Cl.: C08F 292/00, C08K 9/10, G01N 33/543

(54) **PROCEDE DE PREPARATION DE PARTICULES COMPOSITES, PARTICULES COMPOSITES OBTENUES ET LEUR UTILISATION DANS UN TEST DIAGNOSTIC**
VERFAHREN ZUR HERSTELLUNG VON VERBUNDTEILCHEN, ERHALTENE VERBUNDTEILCHEN UND IHRE VERWENDUNG BEI EINEM DIAGNOSTISCHEN TEST
PROCESS FOR PREPARING COMPOSITE PARTICLES, COMPOSITE PARTICLES OBTAINED, AND THEIR USE IN A DIAGNOSTIC TEST

(30) Priorité: 15.12.2005 FR 0553879
(43) Date de publication de la demande: 10.09.2008
(73) Titulaire: BIOMERIEUX, 69280 Marcy-L'Etoile (FR); CENTRE NATIONAL DE LA RECHERCHE SCIENTIFIQUE (CNRS), 75016 Paris (FR)
(72) Inventeur: BRACONNOT, Sébastien, F-92600 Asnières Sur Seine (FR); ELAISSARI, Abdelhamid, F-69230 Saint-Genis-Laval (FR); MOUAZIZ, Hanna, F-72000 Le Mans (FR)
(74) Mandataire: Kopff, Hélène
(86) Numéro de dépôt international: PCT/FR2006/051355
(87) Numéro de publication internationale: WO 2007/068859

(56) Documents cités:
- EP-A1- 0 390 634
- FR-A- 2 800 636
- US-A- 5 356 713
- US-B1- 6 521 341
- US-B1- 6 866 838

## Description

La présente invention concerne le domaine des particules composites., Plus particulièrement, la présente invention a pour objet un procédé de préparation de particules composites isodisperses, stables dans un solvant organique, ayant une diamètre de 50 à 1000 nm par encapsulation par polymérisation d'émulsion appauvrie en phase organique ladite émulsion comprenant des gouttelettes d'émulsion inorganique comprenant une phase organique et des nanoparticules inorganiques distribuées dans ladite phase organique, ledit procédé étant caractérisé en ce que la polymérisation est mise en oeuvre en utilisant, à titre de monomère de polymérisation, de 60 à 100% d'au moins un agent de réticulation et de 0 à 40% d'au moins un monomère hydrophobe, étant entendu qu'au moins 95% du ou des agents de réticulation sont hydrophobes, ainsi que les nouvelles particules composites isodisperses, stables dans un solvant organique, ayant une diamètre de 50 à 1000 nm ainsi obtenues.

L'encapsulation est un procédé utilisé pour l'obtention de particules solides enrobées par au moins une couche de polymère. Un tel système est supposé présenter des propriétés différentes de la somme des propriétés des composants individuels, en particulier de meilleures propriétés mécaniques. Les procédés d'encapsulation ont notamment été utilisé dans le domaine de la préparation de pigments, d'encres, de plastiques et de peintures. Une des applications les plus importantes de particules et pigments encapsulés est trouvée dans le domaine des peintures en émulsion. Mais quand les particules inorganiques obtenues par encapsulation sont magnétisables, cela ouvre des voies particulières dans le domaine de la biologie, par exemple grâce au couplage de protéines ou d'anticorps sur les particules encapsulées pour une utilisation dans des tests de diagnostic. De telles particules sont également utilisées dans des procédés de séparation biochimique. D'une manière générale, les particules encapsulées présentent un intérêt comme support, vecteur ou véhicule dans les domaines de l'ingénierie biologique, du diagnostic et de la pharmacie. À cet effet, elles ont été utilisées dans le diagnostic médical comme support solide pour des macromolécules biologiques.

Les particules colloïdales présentent plusieurs avantages par rapport aux supports solides traditionnels, tels que tubes et plaques. En effet, elles permettent de disposer d'une grande surface pour des interactions, elles diffusent dans le volume, ce qui augmente la cinétique de capture, et elles sont facilement modifiables chimiquement par introduction de groupements fonctionnels susceptibles de réagir avec d'autres molécules ou macromolécules naturelles, synthétiques ou biologiques, telles que des anticorps ou des fragments d'anticorps, des protéines, des polypeptides, des polynucléotides, des acides nucléiques, des fragments d'acides nucléiques, des enzymes, ou des molécules chimiques telles des catalyseurs, des molécules cages, des chélatants, voire des colloïdes biologiques, tels que des bactéries, des cellules, des virus et des parasites.

Parmi les particules colloïdales, les latex magnétisables ont suscité un grand intérêt dans le domaine analytique et sont utilisés par exemple comme moyen pour séparer, concentrer et/ou détecter des analytes, tels que des antigènes, des anticorps, des molécules biochimiques, des acides nucléiques, les bactéries, les virus, les parasites et autres.

Il est possible de classer les particules colloïdales en trois catégories : les petites particules ayant un diamètre inférieur à 50 nm, les grosses particules ayant un diamètre strictement supérieur à 1000 nm et les particules intermédiaires d'un diamètre compris entre 50 et 1000 nm.

Pour que les particules magnétiques soient considérées comme de bons candidats, en particulier pour une application diagnostique, elles doivent répondre à certains critères. Une bonne teneur en charge inorganique (i.e. oxyde de fer), il est préférable que la charge magnétique soit répartie de façon relativement homogène dans le coeur et d'une particule à l'autre. La charge magnétique doit être parfaitement encapsulée afin d'éviter le relargage de la charge magnétique. Elles ne doivent pas s'agréger de manière irréversible sous l'action d'un champ magnétique, ce qui signifie qu'elles puissent être redispersées facilement, rapidement une fois le champ magnétique supprimé.

De même, elles doivent présenter une densité physique relativement faible pour réduire le phénomène de sédimentation. Avantageusement, elles doivent présenter une distribution granulométrique étroite pour une séparation homogène sous l'action d'un champ magnétique. On parle encore de particules monodisperses ou isodisperses.

Ainsi, en raison de leur taille et de leur densité, les grosses particules magnétiques en suspension dans une phase liquide ont tendance à sédimenter rapidement.

A contrario, les petites particules magnétiques ont tendance à rester en suspension du fait de leur mouvement Brownien induit par l'agitation thermique et sont difficilement séparables (voire pas du tout) par un aimant, en particulier si le champ magnétique appliqué est relativement faible. Elles ne sont donc pas bien appropriées pour les utilisations ci-dessus.

Il existe donc un intérêt évident à produire des particules composites présentant une taille intermédiaire entre 50 et 1000 nm qui à la fois pallient les inconvénients précités et répondent notamment aux critères établis ci-dessus. Mais l'invention n'est pas limitée à des particules composites magnétisables, comme décrit ci-après.

La demande de brevet EP-A-0 390 634 décrit des microsphères composites magnétisables de polymère vinylaromatique réticulé hydrophobe d'un diamètre de l'ordre de 50 à 10 000 nm et comprenant un coeur solide constitué de particules magnétisables et d'une écorce constituée d'un copolymère hydrophobe dérivé d'au moins un monomère vinylaromatique hydrophobe et d'au moins un polymère émulsifiant polyéthyléniquement insaturé soluble dans le ou les monomères vinylaromatiques et susceptible de réticuler avec le ou lesdits monomères, Toutefois, bien qu'elles puissent répondre à l'exigence de la taille, les particules présentent l'inconvénient de ne pas avoir une répartition homogène de la charge magnétique qui est localisée à l'intérieur du coeur. Par ailleurs, et comme cela ressort à l'évidence des figures annexées, les particules ne sont pas homogènes en taille. Il s'agit donc d'un ensemble de particules polydisperses qui devront être triées par application d'un champ magnétique pour ne retenir que les particules de taille souhaitées.

On peut également citer les particules Dynabeads (nom commercial, Invitrogen). Ces particules sont des microsphères constituées d'un coeur poreux de polystyrène et d'oxydes de fer. Les oxydes sont déposés dans les pores disponibles par précipitation des sels ferreux et ferriques en milieu basique. Afin d'éviter le relargage des oxydes de fer, l'encapsulation est réalisée par introduction d'une enveloppe d'un autre polymère. Les particules élaborées via ce procédé présentent un diamètre respectivement de 2,8 µm (particules M280) et de 4,5 µm (particules M450). Elles sont considérées comme des particules isodisperses, mais, en raison de leur taille élevée, présentent les inconvénients précités, principalement le phénomène de sédimentation. De plus, leur surface spécifique est faible.

La demande de brevet WO01/33223, déposée par l'une des Demanderesses, décrit des nanosphères composites de diamètre compris entre 50 et 1000 nm, comprenant un coeur essentiellement liquide constitué d'une phase organique et de nanoparticules inorganiques distribuées à l'intérieur de la phase organique, et une enveloppe constituée d'un polymère hydrophile issu de la polymérisation d'au moins un monomère hydrosoluble. Ces particules sont resserrées en taille, de la taille appropriée, mais ont pour inconvénient qu'elles ne sont pas stables dans des solvants organiques.

La demande de brevet WO03/004559, également déposée par l'une des Demanderesses, décrit des particules composites comprenant un coeur en un polymère hydrophobe formant la matrice et des nanoparticules inorganiques distribuées dans ladite matrice, ledit coeur étant au moins entouré par un copolymère amphiphile pour la stabilisation du coeur et permettant d'introduire des fonctions réactives à la surface des particules. Comme précédemment, ces particules sont resserrées en taille, de la taille appropriée, mais ont pour inconvénient qu'elles ne sont pas stables dans des solvants organiques.

Selon l'invention, on dispose maintenant contre toute attente de nouvelles particules composites permettant de pallier tous les inconvénients cités précédemment et qui répondent aux critères suivants :
- particules sphériques très resserrées en taille, voire isodisperses,
- particules ayant une morphologie homogène tendant vers une structure du type coeur/écorce (coeur entouré d'une écorce polymère),
- particules possédant une charge inorganique répartie de manière homogène dans le coeur,
- particules de taille intermédiaire, à savoir ayant un diamètre compris entre 50 et 1000 nm, et de préférence entre 100 et 1000 nm
- particules fonctionnalisées ou fonctionnalisables,
- leur charge inorganique peut être magnétique ou magnétisable,
- particules obtenues par un procédé de synthèse original, simple et contrôlable,
- particules stables dans des solvants organiques, notamment polaires tels que le DMSO (diméthylsulfoxyde), DMF (diméthylformamide), l'acétonitrile et les alcools.

Ainsi, la présente invention a pour objet un procédé de préparation de particules composites isodisperses stables dans un solvant organique, ayant une diamètre de 50 à 1000 nm par encapsulation par polymérisation d'émulsion appauvrie en phase organique ladite émulsion comprenant des gouttelettes d'émulsion inorganique comprenant une phase organique et des nanoparticules inorganiques distribuées dans ladite phase organique, ledit procédé étant caractérisé en ce que la polymérisation est mise en oeuvre en utilisant, à titre de monomère de polymérisation, de 60 à 100% d'au moins un agent de réticulation et de 0 à 40% d'au moins un monomère hydrophobe, étant entendu qu'au moins 95% du ou des agents de réticulation sont hydrophobes.

Les particules composites isodisperses stables dans un solvant organique, ayant une diamètre de 50 à 1000 nm obtenues par le procédé de l'invention sont nouvelles et constituent un autre objet de l'invention.

Enfin, l'invention a également pour objet l'utilisation de ces nouvelles particules composites isodisperses stables dans un solvant organique, ayant une diamètre de 50 à 1000 nm dans des applications biomédicales et en particulier diagnostiques.

Les inventeurs ont donc mis au point pour la première fois un nouveau procédé de préparation de nouvelles générations de particules qui sont, contre toute attente, très stables en milieu aqueux dans lequel elles sont préparées mais également de plus en plus stables à très stables dans les solvants organiques dans lesquels elles sont transférées ou transférables, l'augmentation de la stabilité étant fonction de l'utilisation croissante d'agent de réticulation hydrophobe. De plus, le procédé de l'invention permet d'élaborer des particules composites isodispères, stables dans un solvant organique ayant une morphologie homogène tendant vers une structure du type coeur/écorce, cette dernière structure est conservée même lorsqu'on utilise 100% d'agent de réticulation hydrophobe à titre de monomère de polymérisation. L'avantage d'une telle structure avec une écorce polymère est que celle-ci encapsule parfaitement la charge inorganique.

La caractéristique du procédé réside dans le fait d'utiliser majoritairement (au moins 95% de 60 à 100%) au moins un agent de réticulation hydrophobe comme monomère principal en présence d'émulsion appauvrie en phase organique.

Par émulsion appauvrie en phase organique, on entend une émulsion comprenant une phase inorganique dispersée dans une phase organique, ladite phase organique représentant de 10 mg à 200 mg par gramme d'émulsion sèche, de préférence au plus 100 mg et de préférence encore au plus 50 mg par gramme d'émulsion sèche.

Comme indiqué précédemment, l'augmentation de la quantité d'agent de réticulation hydrophobe à titre de monomère de polymérisation dans le procédé de l'invention permet d'améliorer la stabilité dans les solvants organiques des particules composites obtenues par ledit procédé et d'améliorer leur structure eu égard notamment à leur homogénéité.

Ainsi, le procédé de l'invention utilise au moins 70%, de préférence au moins 80%, de préférence encore au moins 90% d'au moins un agent de réticulation. On préfère encore davantage utiliser au moins 95% et même 100% d'au moins un agent de réticulation, ce qui constitue un mode de réalisation de l'invention. On préfère également utiliser 100% d'agent de réticulation hydrophobe.

Le procédé de l'invention peut mettre en oeuvre un ou plusieurs agents de réticulation dans la mesure où le ou les agents de réticulation majoritaires (au moins 95%) sont hydrophobes.

Par agent de réticulation hydrophobe, on entend toute molécule ou macromolécule possédant au moins deux liaisons vinyliques et présentant une solubilité dans l'eau inférieure à 0,3g/l.

Des exemples d'agent de réticulation hydrophobe comprennent les agents de réticulation à base de dérivé styrénique, tel que le divinylbenzène, et ses dérivés et tout agent de réticulation présentant des groupements hydrophobes.

Selon un mode de réalisation de l'invention, le procédé met en oeuvre au moins un agent de réticulation, de préférence au moins un desdits agents de réticulation est un agent de réticulation à base de dérivé styrénique. Avantageusement, l'agent de réticulation est le divinylbenzène.

Selon un autre mode de réalisation de l'invention, le procédé met en oeuvre un seul agent de réticulation, de préférence un agent de réticulation à base de dérivé styrénique, et de préférence le divinylbenzène.

Lorsque le procédé met en oeuvre au moins deux agents de réticulation, et de préférence deux agents de réticulation, au moins l'un des deux est un agent de réticulation fluorescent ou bien un agent de réticulation hydrophile.

Les agents de réticulation hydrophiles (non hydrophobes) comprennent les dérivés acrylamides, tels que le méthylènebisacrylamide et l'éthylèneglycodiméthacrylate, et tout agent de réticulation présentant des groupements hydrosolubles.

A titre d'agent de réticulation fluorescent, on peut citer les dérivés du pyrène, les dérivés de la fluorescéine portant à ses extrémités deux fonctions méthacrylates, comme fluorescéinediméthacrylate (Polyfluor© 511), le naphtalènediméthacrylate. De tels agents de réticulation fluorescents sont disponibles chez Polysciences (US coporation).

Selon un mode de réalisation particulier de l'invention, le procédé met en oeuvre au plus 5% d'un agent de réticulation hydrophile, de préférence à base d'acrylamide, ou d'un agent de réticulation fluorescent.

L'ajout d'un agent de réticulation fluorescent dans le procédé de l'invention permet d'obtenir des particules fluorescentes en une seule étape et leur utilisation ultérieure dans des applications où un marquage est approprié, comme dans les applications biomédicales telles que le diagnostic.

L'ajout d'un agent de réticulation hydrophile, notamment à base d'acrylamide, dans le procédé de l'invention permet l'obtention de particules de surface hydrophile en une seule étape et leur utilisation ultérieure dans des applications où une liaison avec un partenaire de liaison est approprié, comme dans les applications biomédicales telles que le diagnostic.

Selon un mode de réalisation de l'invention, le procédé de l'invention met en oeuvre deux agents de réticulation, l'un étant un agent de réticulation hydrophobe, de préférence le divinylbenzène, en quantité majoritaire, et l'autre étant un agent de réticulation fluorescent en quantité minoritaire, de préférence à raison de 1%, ou bien l'un étant un agent de réticulation à base de dérivé styrénique, de préférence le divinylbenzène, en quantité majoritaire, et l'autre étant un agent de réticulation hydrosoluble (à base d'acrylamide) en quantité minoritaire, de préférence à raison de 5%.

Le procédé de l'invention met également en oeuvre au plus 40% d'au moins un monomère hydrophobe.

Par monomère hydrophobe, on entend un monomère présentant une seule liaison vinylique et une solubilité dans l'eau inférieure à 0,5 g/l.

Des exemples de tels monomères comprennent les monomères styréniques ou dérivés, le styrène étant particulièrement préférés.

Lorsque dans le procédé de l'invention, on n' utilise ni un agent de réticulation fluorescent, ni un agent de réticulation hydrophile, on peut utiliser un monomère fluorescent en quantité minoritaire pour marquer les particules.

Ainsi, selon un mode de réalisation, le procédé de l'invention met en oeuvre de 0 à 30 et de préférence de 0 à 35% d'un monomère hydrophobe, notamment styrénique, et au plus 10 et de préférence 5% d'un monomère hydrophobe fluorescent.

Les monomères fluorescents hydrophobes sont largement connus de l'homme du métier.

A titre de monomère fluorescent hydrophobe, on peut citer le 2-naphtylmethacrylate (polyfluor 345), monomère fluorescent utilisé en très faible quantité pour fonctionnaliser les particules.

Ainsi, dans le procédé de l'invention, on peut utiliser différentes formulations de monomères, étant entendu qu'on a toujours une quantité majoritaire d'agent de réticulation hydrophobe et qu'on n'a jamais en même temps une quantité minoritaire (d'au plus 10 et de préférence 5 %) soit de monomère hydrophobe fluorescent, soit d'agent de réticulation fluorescent, soit d'agent de réticulation hydrophile.

Des exemples non limitatifs de formulations de monomères comprennent :
- 60% à 99 % d'un agent de réticulation hydrophobe, en particulier du divinylbenzène, et 1 à 40% d'un monomère hydrophobe, en particulier le styrène ;
- 60 à 99,9% d'un agent de réticulation hydrophobe, en particulier du divinylbenzène, 39,9 à 0% d'un monomère hydrophobe, en particulier le styrène, et 0,1 à 10%, de préférence 5%, soit de monomère fluorescent, soit d'agent de réticulation fluorescent, soit d'agent de réticulation hydrophile.

Des exemples de phase organique dans laquelle les nanoparticules inorganiques sont distribuées, comprennent les phases comprenant un hydrocarbure aliphatique, cyclique ou aromatique. En particulier, l'hydrocarbure est choisi parmi les alcanes, de préférence ayant au moins 5 atomes de carbone, tels que le pentane, l'hexane, l'heptane, l'octane, le nonane, le décane, l'undécane, le dodécane, l'octane étant particulièrement préféré.

Les nanoparticules inorganiques appropriées aux fins de l'invention sont toutes nanoparticules ayant un diamètre de particules d'au plus 50 nm, de préférence d'au plus 10 nm et de préférence encore d'au moins 1 nm.

Selon un mode de réalisation, lesdites nanoparticules sont choisies parmi les oxydes métalliques de fer, de titane, de cobalt, de zinc, de cuivre, de manganèse, de nickel ; la magnétite ; l'hématite ; les ferrites tels que les ferrites de manganèse, nickel, manganèse-zinc ; les alliages de cobalt, nickel ; les zéolites ; le talc ; les argiles telles que la bentonite et kaolin ; l'alumine ; la silice ; le graphite ; les cristaux fluorescents (comme par exemple CdSe) ; l'or colloïdale ; et le noir de carbone.

Selon un autre mode de réalisation, lesdites nanoparticules inorganiques sont des nanoparticules magnétiques et elles sont choisies parmi les oxydes métalliques et de préférence les oxydes de fer comme la magnétite et la maghémite.

Lesdites nanoparticules inorganiques sont présentes dans l'émulsion à raison de 40 à 80% et de préférence à raison de 60%.

La taille de l'émulsion est comprise entre 150 et 300 nm. De préférence, elle est inférieure à 250 nm et de préférence encore supérieure à 180 nm.

La préparation de l'émulsion utile aux fins de l'invention peut être mise en oeuvre par toute méthode connue de l'homme du métier. Un exemple de tel procédé est décrit par J. Bibette, 1993, J. Magn. Magn. Mater, 122 : 37 ou dans la demande de brevet WO01/33223.

Le procédé d'encapsulation par polymérisation d'émulsion est largement connu de l'homme du métier et est décrit par exemple dans la demande de brevet WO01/33223.

Selon un mode de réalisation préféré, le procédé de préparation de particules composites de l'invention comprend ou consiste en les étapes consistant à :
(a) mettre en présence l'émulsion avec un tensioactif,
(b) ajouter un ou des agents de réticulation hydrophobes et
(c) procéder à la polymérisation

Les agents tensioactifs sont largement connus de l'homme du métier. Selon un mode de réalisation approprié, ils sont choisis parmi les tensioactifs anioniques tels que les polymères amphiphiles et le dodécylsulfate de sodium, et les tensioactifs non ioniques tels que le triton X-405, les tensioactifs anioniques tels que les polymères amphiphiles et le dodécylesulfate de sodium étant particulièrement préférés.

Les polymères amphiphiles appropriés aux fins de l'invention sont par exemple décrits dans la demande de brevet EP 892 020. Des exemples de tels polymères sont disponibles auprès de la société Coatex SA (France).

Comme indiqué précédemment, le principe de la polymérisation est largement connu de l'homme du métier. Elle est amorcée pour tout procédé d'amorçage connu de l'homme du métier tel que par élévation de la température pour favoriser le décomposition et/ou en présence d'un amorceur radicalaire, ou par photochimie à l'aide de rayonnements, tels que des rayonnements UV ou un faisceau laser ou d'autres sources d'énergie.

Selon un mode de réalisation de l'invention, la polymérisation est mise en oeuvre en présence d'un amorceur, de préférence un amorceur radicalaire hydrosoluble.

Des exemples d'amorceurs hydrosolubles comprennent par exemple le persulfate de potassium et le métabisulfite, le persulfate de potassium étant particulièrement préféré.

L'amorceur est introduit soit simultanément à l'introduction des agents de réticulation, soit préalablement à leur introduction, soit encore postérieurement à leur introduction.

La polymérisation est mise en oeuvre de préférence par élévation de la température jusqu'à environ 60 à environ 90°C, de préférence à environ 70°C, en présence de l'amorceur de polymérisation, étant entendu que les conditions de polymérisation seront déterminées par l'homme du métier en fonction de l'amorçage choisi.

Les particules composites obtenues par le procédé de l'invention sont nouvelles, de sorte que l'invention a également pour objet les particules composites susceptibles d'être obtenues par le procédé décrit précédemment.

Les nouvelles particules composites de l'invention comprennent une matrice polymère réticulée dans laquelle sont distribuées des nanoparticules inorganiques.

La matrice polymère réticulée est obtenue à partir d'au moins un agent de réticulation comme défini précédemment.

Les nanoparticules inorganiques proviennent de l'émulsion et représentent de 40 à 80% en masse par rapport à la masse totale de l'émulsion à l'état sec.

Les particules composites de l'invention trouvent notamment des applications dans le domaine de la peinture, des encres, des plastiques et, quand elles sont fonctionnalisées, dans des domaines divers de la biologie, en particulier pour la séparation de molécules biologiques ou biochimiques, pour des essais de diagnostic, pour le tri cellulaire, pour la capture biologique ou biochimique, pour des applications analytiques et également pour la chimie supportée.

Selon un mode de réalisation de l'invention, les particules composites présentent de plus à leur surface des groupements fonctionnels réactifs susceptibles de réagir avec au moins un ligand ou un polymère.

Par ligand, on entend une molécule biologique telle qu'un anticorps, un fragment d'anticorps, une protéine, un polypeptide, une enzyme, un polynucléotide, une sonde, une amorce, un fragment d'acide nucléique ; une molécule chimique telle que des polymères chimiques, des substances médicamenteuses, des molécules cages, des agents chélatants, des catalyseurs.

Des exemples de groupements fonctionnels appropriés aux fins de l'invention comprennent les groupements carboxylique, amine, thiol, aldéhyde, hydroxyle, tosyle, hydrazine l'acide phenyl-boronique et des esters activés.

Ces groupements fonctionnels peuvent être apportés par un traitement de la surface des particules, par exemple par traitement chimique tel qu'hydrolyse ou greffage, ou bien par introduction d'agent de réticulation fonctionnalisé ou selon des procédés (chimique ou physique) largement connus de l'homme du métier

Selon un autre mode de réalisation de l'invention, les particules présentent de plus un agent de liaison spécifique pour une substance susceptible de liaison.

Par agent de liaison spécifique pour une substance susceptible de liaison, on entend un composé intermédiaire qui se lie à un substance qui est susceptible de liaison à un ligand. A titre de couple agent de liaison spécifique/substance susceptible de liaison, on peut citer les couples biotine/streptavidine, haptène/anticorps, antigène/anticorps, peptide/anticorps, sucre/lectine, polynucléotide/complémentaire du polynucléotide.

Selon un mode de réalisation particulier de l'invention, l'agent de liaison spécifique est la streptavidine.

Les particules de l'invention peuvent également présenter de plus à leur surface une couche hydrophile fonctionnalisée pour une liaison ultérieure à un ligand, ce qui constitue un autre mode de réalisation de l'invention.

A titre de couche hydrophile fonctionnalisée, on peut citer les couches de polymères hydrophiles naturels ou synthétiques, telles qu'une couche de dextrane, de chitosane, de derivés poly(N-alkylacrylamide), poly(N-alkylmethacrylamide), poly(acide acrylique), poly(oxyde d'éthylene).

La couche hydrophile peut être appliquée aux particules de l'invention par adsorption ou liaison covalente selon des procédés largement connus de l'homme du métier.

Les particules ainsi modifiées et liées à un ligand sont également nouvelles.

Ainsi, l'invention a également pour objet des conjugués comprenant une particule modifiée telle que définie précédemment et un ligand, ledit ligand pouvant être lié à une substance susceptible de liaison.

Les conjugués trouvent également des applications dans des domaines divers de la biologie, en particulier pour la séparation de molécules biologiques ou biochimiques, pour des essais de diagnostic, pour le tri cellulaire, pour la capture de bactéries, pour la capture biologique ou biochimique.

Ainsi, un autre objet de l'invention concerne l'utilisation des particules ou des conjugués de l'invention dans un test de diagnostic *in vitro.*

A titre de diagnostic *in vitro*, on peut citer les tests de dosage des acides nucléiques et dérivés, des protéines et dérivés, et des cellules et dérivés.

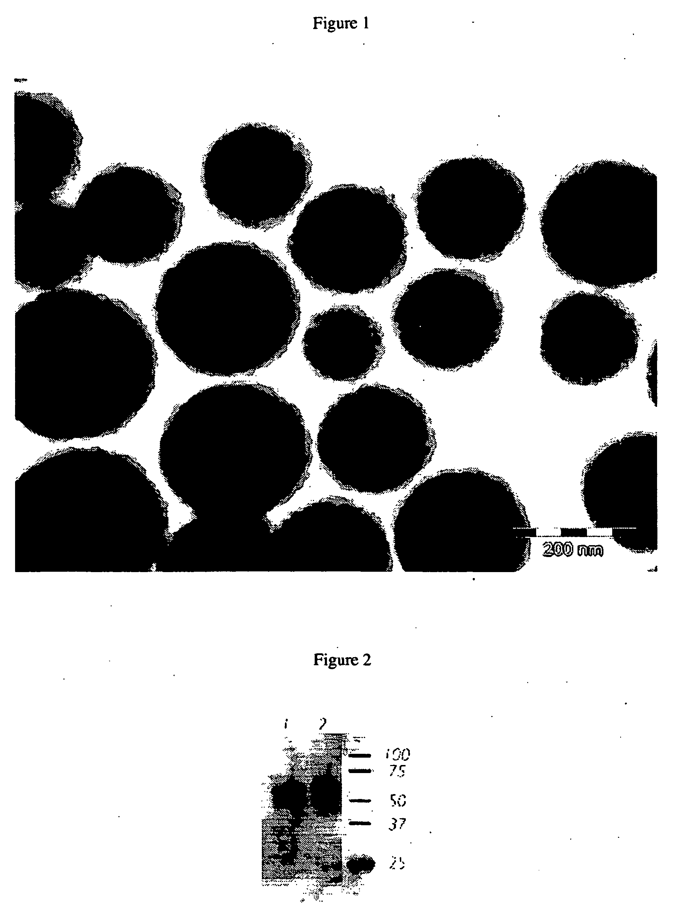

L'invention sera mieux comprise à l'aide des exemples suivants donnés uniquement à titre illustratif et non-limitatif, ainsi qu'à l'aide des figures 1 et 2 sur lesquelles :
- la figure 1 est une photographie de microscopie électronique des particules de l'invention préparées avec 100% d'agent de réticulation hydrophobe, et
- la figure 2 est une photographie de Western-blot mettant en évidence la capture de protéine transmembranaire (TM) issue d'un lysat cellulaire par les particules composites de l'invention sur lesquelles sont greffés des anticorps anti-TM, la piste 1 correspondant à des particules auxquelles ont été ajoutés 50 µg d'anticorps, la piste 2 correspondant à des particules auxquelles ont été ajoutés 150 µg d'anticorps, la piste de droite correspondant à la piste des marqueurs de poids moléculaire.

### Exemple 1 : Préparation de particules de l'invention

Les polymérisations sont effectuées dans un réacteur en verre d'une contenance de 50 ml muni d'un système d'agitation mécanique (ancre en Téflon, forme en demi-lune). Ce réacteur est équipé d'un réfrigérant et possède une double enveloppe permettant la circulation d'eau provenant d'un bain thermostaté.

Les polymérisations sont réalisées en présence de 50 ml d'émulsion magnétique présentant un taux de solide de 4% (soit 2 g d'émulsion) (Ademtech, émulsion préparée selon le protocole décrit dans la demande de brevet WO01/33223, ladite émulsion présentant de 10 à 250 mg d'octane, de 20 à 30% de tensioactifs et de 70 à 80% d'oxyde de fer, leur diamètre de particule étant de 180 à 270 nm). L'émulsion est préalablement « lavée » trois fois avec une solution aqueuse contenant un agent tensioactif (polymère amphiphile Coatex M883 à 0,5 g/l préparé à pH 9), puis une dernière fois avec de l'eau déionisée, bouillie et dégazée, avant d'être finalement introduite dans le réacteur de polymérisation. L'opération de «lavage » de l'émulsion consiste à séparer magnétiquement les gouttelettes de la phase dispersante à l'aide d'un aimant et de remplacer la phase continue par une nouvelle à volume égal. Le milieu réactionnel est ensuite maintenu sous atmosphère inerte (quelques minutes sous un flux régulier de diazote N₂).

L'agent de réticulation hydrophobe divinylbenzène (DVB) (900 mg) est ajouté à l'émulsion magnétique placée sous agitation afin qu'il puisse aisément diffuser dans les gouttelettes d'émulsion pendant une heure. Le persulfate de potassium (KPS) (18mg) est ajouté après l'étape de diffusion et lorsque le milieu réactionnel est à température de polymérisation. Les polymérisations sont effectuées à une température de 70°C et pendant 20 heures.

Les particules obtenues sont sphériques, isodisperses, elles ont une morphologie homogène du type coeur/écorce, elles possèdent une charge inorganique répartie de manière homogène dans le coeur, elles sont de taille intermédiaire, à savoir ayant un diamètre compris entre 50 et 1000 nm, comme montré sur la figure 1 qui est une photographie de microscopie électronique prise après séchage à l'air libre d'une goutte des particules obtenues.

Par ailleurs, ces particules ont une charge inorganique magnétique ou magnétisable, elles sont fonctionnalisées ou fonctionnalisables (voir exemple 6 ci-après) et elles sont obtenues par un procédé de synthèse original, simple et contrôlable.

### Exemple 2 : Préparation de particules de l'invention

On a procédé comme indiqué dans l'exemple 1 ci-dessus, à ceci près qu'on a utilisé 1500 mg de DVB.

### Exemple 3 : Préparation de particules de l'invention

Les polymérisations sont effectuées dans un réacteur en verre d'une contenance de 50 ml muni d'un système d'agitation mécanique (ancre en Téflon, forme en demi-lune). Ce réacteur est équipé d'un réfrigérant et possède une double enveloppe permettant la circulation d'eau provenant d'un bain thermostaté.

Les polymérisations sont réalisées en présence de 50 ml d'émulsion magnétique présentant un taux de solide de 4% (soit 2 g d'émulsion) (Ademtech, *supra*). L'émulsion est préalablement « lavée » trois fois avec une solution aqueuse contenant un agent tensioactif (polymère amphiphile Coatex M883 à 0,5 g/l préparé à pH 9), puis une dernière fois avec de l'eau déionisée, bouillie et dégazée, avant d'être finalement introduite dans le réacteur de polymérisation. L'opération de « lavage » de l'émulsion consiste à séparer magnétiquement les gouttelettes de la phase dispersante à l'aide d'un aimant et de remplacer la phase continue par une nouvelle à volume égal. Le milieu réactionnel est ensuite maintenu sous atmosphère inerte (quelques minutes sous un flux régulier de diazote N₂).

L'agent de réticulation DVB (900 mg) et l'agent de réticulation fluorescent polyFluor 511 (1% en masse par rapport au DVB soit 9 mg) sont ajoutés à l'émulsion magnétique placée sous agitation afin qu'il puisse aisément diffuser dans les gouttelettes d'émulsion pendant une heure. Le persulfate de potassium (KPS) (18mg) est ajouté après l'étape de diffusion et lorsque le milieu réactionnel est à température de polymérisation. Les polymérisations sont effectuées à une température de 70°C et pendant 20 heures.

### Exemple 4 : Préparation de particules de l'invention

Les polymérisations sont effectuées dans un réacteur en verre d'une contenance de 50 ml muni d'un système d'agitation mécanique (ancre en Téflon, forme en demi-lune). Ce réacteur est équipé d'un réfrigérant et possède une double enveloppe permettant la circulation d'eau provenant d'un bain thermostaté.

Les polymérisations sont réalisées en présence de 50 ml d'émulsion magnétique présentant un taux de solide de 4% (soit 2 g d'émulsion) (Ademtech, *supra*). L'émulsion est préalablement « lavée » trois fois avec une solution aqueuse contenant un agent tensioactif (polymère amphiphile Coatex M883 à 0,5 g/l préparé à pH 9), puis une dernière fois avec de l'eau déionisée, bouillie et dégazée, avant d'être finalement introduite dans le réacteur de polymérisation. L'opération de « lavage » de l'émulsion consiste à séparer magnétiquement les gouttelettes de la phase dispersante à l'aide d'un aimant et de remplacer la phase continue par une nouvelle à volume égal. Le milieu réactionnel est ensuite maintenu sous atmosphère inerte (quelques minutes sous un flux régulier de diazote N₂).

L'agent de réticulation DVB (900 mg) et un monomère fluorescent Polyfluor 345 (2% en masse par rapport au DVB soit 18 mg) sont ajoutés à l'émulsion magnétique placée sous agitation afin qu'il puisse aisément diffuser dans les gouttelettes d'émulsion pendant une heure. Le persulfate de potassium (KPS) (18mg) est ajouté après l'étape de diffusion et lorsque le milieu réactionnel est à température de polymérisation. Les polymérisations sont effectuées à une température de 70°C et pendant 20 heures.

### Exemple 5 : Préparation de particules de l'invention

Les polymérisations sont effectuées dans un réacteur en verre d'une contenance de 50 ml muni d'un système d'agitation mécanique (ancre en Téflon, forme en demi-lune). Ce réacteur est équipé d'un réfrigérant et possède une double enveloppe permettant la circulation d'eau provenant d'un bain thermostaté.

Les polymérisations sont réalisées en présence de 50 ml d'émulsion magnétique présentant un taux de solide de 4% (soit 2 g d'émulsion) (Ademtech, *supra*). L'émulsion est préalablement « lavée » trois fois avec une solution aqueuse contenant un agent tensioactif (polymère amphiphile Coatex M883 à 0,5 g/l préparé à pH 9), puis une dernière fois avec de l'eau déionisée, bouillie et dégazée, avant d'être finalement introduite dans le réacteur de polymérisation. L'opération de « lavage » de l'émulsion consiste à séparer magnétiquement les gouttelettes de la phase dispersante à l'aide d'un aimant et de remplacer la phase continue par une nouvelle à volume égal. Le milieu réactionnel est ensuite maintenu sous atmosphère inerte (quelques minutes sous un flux régulier de diazote N₂).

L'agent de réticulation DVB (900 mg) et l'agent de réticulation hydrosoluble methylène bis acrylamide (MBA) (5% en masse par rapport au DVB soit 45 mg) sont ajoutés à l'émulsion magnétique placée sous agitation afin qu'il puisse aisément diffuser dans les gouttelettes d'émulsion pendant une heure. Le persulfate de potassium (KPS) (18mg) est ajouté après l'étape de diffusion et lorsque le milieu réactionnel est à température de polymérisation. Les polymérisations sont effectuées à une température de 70°C et pendant 20 heures.

### Exemple 6: Fonctionnalisation des particules composites élaborées par fixation d'un polymère hydrophile aminé.

### 6.1. Via adsorption (processus physique)

Deux ml des particules magnétiques obtenues dans l'exemple 1 ci-dessus sont lavés trois fois avec une solution de Triton X-405 à 1 g/l. Les particules lavées sont reprises dans 1,9 ml de tampon acétate (0,002 M, pH 5,6). 100 µl d'une solution d'aminodextrane (de concentration variable de 15 à 160 g/l) sont ajoutés aux particules. Le mélange est homogénéisé pendant une nuit. Les particules sont par la suite séparées sous l'action d'un champ magnétique et lavées avec le tampon acétate (0,002 M, pH 5,6).

### 6.2. Via réaction chimique

Deux ml des particules magnétiques obtenues dans l'exemple 1 ci-dessus sont lavés trois fois avec une solution de Triton X-405 à 1 g/l. Les particules lavées sont reprises dans 1,9 ml de tampon acétate (0,002 M, pH 5,6). 100 µl d'une solution de carboodiimide hydrosoluble 1-(3-dimethylaminopropyl)-3-ethylcarbodiimide hydrochloride (EDAC) sont ajoutés à la dispersion particulaire suivie d'une incubation à température ambiante durant 40 minutes. Les particules sont lavées trois fois avec 1,9 ml du tampon acétate (0,002 M, pH 5,6). 100 µl d'une solution d'aminodextrane (de concentration variable de 15 à 160 g/l) sont ajoutés au particules. Le mélange est homogénéisé pendant une nuit. Les particules sont par la suite séparées sous l'action d'un champ magnétique et lavées avec le tampon acétate (0,002 M, pH 5,6). Après une incubation d'une nuit, les particules magnétiques sont lavées trois fois avec le tampon acétate (0,002 M, pH 5,6).

### Exemple 7. Capture antigénique

### 7.1. Greffage batch avec un carbodiimide organosoluble

Un mg des particules magnétiques obtenues dans l'exemple 1 ci-dessus (soit 25 µl, taux de solide initial des particules : 4,07%) est utilisé. Après une séparation magnétique et élimination du surnageant, le culot est complété jusqu'à 25µl avec du tampon phosphate 50 mM pH 4,4 + Tween 20 à 1%. Cinq µl de dicyclohexylcarbodiimide (DCC) dans DMSO sont ajoutés à 50 mM (10 µl DCC + 190 µl DMSO). Cinq µl de N-hydroxysuccinimide (NHS) dans DMSO à 1,12 M (soit 130 mg/ml) sont ajoutés au mélange, puis 46,5 µl d'anticorps (i.e. 200 µg, concentration initiale en anticorps : 4,3 mg/ml). La dispersion est complétée jusqu'à 500 pl avec du tampon phosphate 50 mM pH 4,4 + Tween 20 à 1%. Après une incubation de 3 h à 37°C (agitation au thermomixer, 1000 rpm), une séparation magnétique et remplacement du surnageant par du tampon phosphate 10 mM à pH 6,8 sont réalisées.

### 7.2. Greffage en deux étapes avec un carbodiimide organosoluble :

Un mg des particules magnétiques obtenues dans l'exemple 1 ci-dessus (soit 25 µl, taux de solide initial des particules : 4,07%) est lavé par séparation magnétique, élimination du surnageant, et redispersé dans 25 µl du tampon phosphate 50 mM pH 4,4 + Tween 20 à 1%. Puis 5 µl de dicyclohexylcarbodiimide (DCC) dans DMSO à 50 mM (10 µl DCC + 190 µl DMSO) sont ajoutés, opération suivie d'un ajout de 5 µl de N-hydroxysuccinimide (NHS) dans DMSO à 1,12 M (soit 130 mg/ml). La dispersion est complétée jusqu'à 500 µl avec du tampon phosphate 50 mM pH 4,4 + Tween 20 à 1%. Le mélange est incubé 30 minutes à température ambiante sur une roue d'agitation. Après une séparation magnétique et élimination du surnageant, 46,5 µl d'anticorps (i.e. 200 µg, concentration initiale en anticorps : 4,3 mg/ml) sont ajoutés et la dispersion est complétée jusqu'à 500 µl avec du tampon phosphate 10 mM pH 6,8. Après une incubation de 3 h à 37°C (agitation au thermomixer, 1000 rpm), les particules magnétiques sont séparées par application de champ magnétique et remplacement du surnageant par du tampon phosphate 10 mM à pH 6,8.

### 7.3. Adsorption d'anticorps

Un mg des particules magnétiques obtenues dans l'exemple 1 ci-dessus (soit 20 µl, taux de solide initial des particules : 4,99%) est lavé par séparation magnétique, élimination du surnageant, et redispersion dans 20 µl du tampon phosphate 10 mM pH 6,8. Puis 46,5 µl d'anticorps (i.e. 200 µg, concentration initiale en anticorps : 4,3 mg/ml) sont ajoutés, et la dispersion est complétée jusqu'à 500 µl avec du tampon phosphate 10 mM pH 6,8. Cette opération est suivie d'une étape d'incubation pendant 3 h à 37°C (agitation au thermomixer, 1000 rpm). Après une séparation magnétique, le surnageant est remplacé par du tampon phosphate 10 mM à pH 6,8.

### 7.4. Greffage et Immunoprécipitation

On utilise 22 µl de particules magnétiques obtenues dans l'exemple 2 ci-dessus à 5,5% en taux de solide + 98 µl de tampon d'activation (Ademtech) : soit 120 µl de particules à 1%. Après une séparation magnétique et élimination du surnageant, les particules sont dispersées dans 120 µl de tampon d'activation (opération répétée deux fois). Puis 96 µl de chlorhydrate de N-(3-Diméthylaminopropyl)-N'-éthylcarbodiimide (EDAC) à 4 mg/ml dans tampon d'activation, soit un volume total de 216 µl, sont ajoutés. Une incubation est réalisée 10 minutes à 40°C (agitation thermomixer à 900 rpm), laquelle est suivie de l'ajout de 50 ou 150 µg d'anticorps anti-TM (6A2B2, bioMérieux, France) à 8,27 mg/ml pour 54 µl de particules activées avec l'EDAC. Après une incubation 2 h à 37°C (agitation thermomixer à 900 rpm), 15 µl de BSA à 2 mg/ml sont ajoutés. Après une dernière incubation à 30 minutes à 40°C (agitation thermomixer à 900 rpm), les particules sont séparées magnétiquement et le surnageant est remplacé par du tampon de stockage (Ademtech)

Trente µl de particules préparés comme ci-dessus sensibilisées avec l'anticorps sont ajoutées à 62 µl de lysat cellulaire (lysat de cellules TELCeb6 transfectées avec une protéine d'enveloppe reconnue par les anticorps anti-TM, Blond JL. et al., 2000, Journal of Virology, 74(7) : 3321-3329) et 338 µl de tampon de lyse (PBS-Triton X-100 à 0,05%). Le mélange est agité toute la nuit (12 heures) à 4°C, opération suivie d'une séparation magnétique pendant deux minutes et d'un lavage avec tampon de lyse.

Les protéines libérées (en utilisant le tampon tris, glycérol, SDS à 1% et 5 % beta-mercapto-éthanol, pendant 5 minutes à 100°C) du culot magnétique sont analysées par Électrophorèse et Western blot.

Les résultats du Western Blot sont montrés sur la figure unique qui est une photographie me ttant en évidence la capture de protéine transmembranaire (TM) issue du lysat cellulaire par les particules de l'invention sur lesquelles sont greffés les anticorps anti-TM soit en utilisant 50 µg d'anticorps (piste 1), soit 150 µg (piste 2), la piste de droite correspondant à la piste des marqueurs de poids moléculaire.

Les résultats mettent en évidence la capture des protéines et donc que l'activité des anticorps n'est pas modifiée suite à son greffage. Par ailleurs, les résultats montrent également qu'on n'observe pas de différence selon la quantité d'anticorps utilisée.

### Exemple 8. Mise en évidence de la stabilité des particules de l'invention

On a utilisé des particules de latex magnétiques obtenues dans l'exemple 6.1, pour lesquelles :
- la taille de l'émulsion magnétique avant polymérisation est de l'ordre de 220 nm,
- après polymérisation, la taille des particules est de l'ordre de 267 nm et
après fonctionnalisation par l'aminodextrane, la taille moyenne est de 325 nm, et on a procédé comme suit :
a) 1 ml de particules de latex magnétiques est séparé sous l'action d'un aimant permanent, le surnageant limpide est supprimé et est remplacé instantanément par 1 ml de DMSO. Après une homogénéisation de quelques minutes, la taille des particules est mesurée en utilisant la diffusion de la lumière (NanoZS de Malvern Instrument). La taille moyenne est de 331 nm.
b) 1 ml de particules de latex magnétiques est séparé sous l'action d'un aimant permanent, le surnageant limpide est supprimé et le culot de particules est séché à l'air libre, soit à température ambiante pendant 48h. Le culot est ensuite redispersé dans 1 ml de DMSO ou dans 1 ml d'eau en utilisant un vortex. La taille moyenne de particules est comprise entre 330 nm et 380 nm.

Les résultats ci-dessus mettent en évidence que les particules de l'invention sont stables et redispersables dans l'eau et également dans les solvants organiques polaires.

## Revendications

1. Procédé de préparation de particules composites isodisperses, stables dans un solvant organique, ayant un diamètre de 50 à 1000 nm, par encapsulation par polymérisation d'émulsion appauvrie en phase organique, ladite émulsion consistant en des gouttelettes d'émulsion inorganique comprenant une phase organique et des nanoparticules inorganiques distribuées dans ladite phase organique, **caractérisé en ce que** la polymérisation est mise en oeuvre en utilisant, à titre de monomère de polymérisation, de 60 à 100% d'au moins un agent de réticulation et de 0 à 40% d'au moins un monomère hydrophobe, étant entendu qu'au moins 95% du ou des agents de réticulation sont hydrophobes.

2. Procédé de préparation de particules composites selon la revendication 1, **caractérisé en ce qu'**il met en oeuvre au moins 95% d'au moins un agent de réticulation.

3. Procédé de préparation de particules composites selon la revendication 1, **caractérisé en ce qu'**il met en oeuvre 100% d'au moins un agent de réticulation hydrophobe.

4. Procédé de préparation de particules composites selon l'une quelconque des revendications 1 à 3, **caractérisé en ce qu'**il met en oeuvre un agent de réticulation à base de dérivés styréniques.

5. Procédé de préparation de particules composites selon la revendication 4, **caractérisé en ce que** l'agent de réticulation est le divinylbenzène.

6. Procédé de préparation de particules composites selon l'une quelconque des revendications précédentes, **caractérisé en ce qu'**il met en oeuvre un seul agent de réticulation.

7. Procédé de préparation de particules composites selon l'une quelconque des revendications précédentes, **caractérisé en ce qu'**il met en oeuvre de 0 à 35% d'un monomère hydrophobe styrénique et au plus 5% d'un monomère hydrophobe fluorescent.

8. Procédé de préparation de particules composites selon l'une quelconque des revendications 1 à 5, **caractérisé en ce qu'**il met en oeuvre au plus 5% d'un agent de réticulation fluorescent ou d'un agent de réticulation à base d'acrylamide.

9. Procédé de préparation de particules composites selon l'une quelconque des revendications précédentes, **caractérisé en ce que** la phase organique comprend un alcane.

10. Procédé de préparation de particules composites selon la revendication 9, **caractérisé en ce que** l'alcane est l'octane.

11. Procédé de préparation de particules composites selon l'une quelconque des revendications précédentes, **caractérisé en ce que** les nanoparticules inorganiques sont choisies parmi les oxydes métalliques de fer, de titane, de cobalt, de zinc, de cuivre, de manganèse, de nickel ; la magnétite ; l'hématite ; les ferrites tels que les ferrites de manganèse, nickel, manganèse-zinc ; les alliages de cobalt, nickel ; les zéolites ; le talc ; les argiles telles que la bentonite et kaolin ; l'alumine ; la silice ; le graphite ; les cristaux fluorescents ; l'or colloïdale ; et le noir de carbone.

12. Procédé de préparation de particules composites par encapsulation par polymérisation d'émulsion selon l'une quelconque des revendications précédentes, **caractérisé en ce qu'**il comprend les étapes consistant à :
(a) mettre en présence l'émulsion avec un tensioactif,
(b) ajouter un ou des agents de réticulation hydrophobe à titre de monomère et
(c) procéder à la polymérisation.

13. Procédé de préparation de particules composites selon la revendication 12, **caractérisé en ce que** la polymérisation est mise en oeuvre en présence d'un amorceur.

14. Procédé de préparation de particules composites selon la revendication 13, **caractérisé en ce que** l'amorceur est un amorceur radicalaire hydrosoluble.

15. Procédé de préparation de particules composites selon la revendication 14, **caractérisé en ce que** l'amorceur radicalaire hydrosoluble est le persulfate de potassium.

16. Procédé de préparation de particules composites selon l'une quelconque des revendications 12 à 15, caractérisé que l'agent tensioactif est un polymère amphiphile ou le dodécylsulfate de sodium.

17. Particules composites isodisperses, stables dans un solvant organique, ayant un diamètre de 50 à 1000 nm, susceptibles d'être obtenues par le procédé tel que défini dans l'une quelconque des revendications précédentes.

18. Particules composites selon la revendication 17, **caractérisées en ce qu'**elles présentent de plus à leur surface des groupements fonctionnels réactifs susceptibles de réagir avec au moins un ligand ou un polymère.

19. Particules composites selon la revendications 17, **caractérisées en ce qu'**elles présentent de plus un agent de liaison spécifique pour une substance susceptible de liaison.

20. Particules composites selon la revendication 19, **caractérisées en ce que** l'agent de liaison spécifique est la streptavidine.

21. Particules composites selon la revendications 17, **caractérisées en ce qu'**elles présentent de plus à leur surface une couche hydrophile fonctionnalisée.

22. Particules composites selon la revendications 21, caractérisées en ce la couche hydrophile fonctionnalisée est constituée de dextrane.

23. Conjugués comprenant une particule telle que définie dans la revendication 18 ou la revendication 21 et un ligand.

24. Conjugués comprenant une particule telle que définie dans la revendication 19 et un ligand lié à une substance susceptible de liaison.

25. Utilisation des particules telles que définies dans les revendications 17 à 22 ou des conjugués tels que définis dans les revendications 23 ou 24 dans un test diagnostic.

## Claims

1. A method for preparing stable isodisperse composite particles in an organic solvent, having a diameter from 50 to 1,000 nm, with encapsulation by polymerization of an emulsion depleted in organic phase, said emulsion consisting in inorganic emulsion droplets comprising an organic phase and inorganic nanoparticles distributed in said organic phase, **characterized in that** polymerization is applied by using, as a polymerization monomer, from 60 to 100% of at least one cross-linking agent and from 0 to 40% of at least one hydrophobic monomer, it being understood that at least 95% of the cross-linking agent(s) are hydrophobic.

2. The method for preparing composite particles according to claim 1, **characterized in that** it applies at least 95% of at least one cross-linking agent.

3. The method for preparing composite particles according to claim 1, **characterized in that** it applies 100% of at least one hydrophobic cross-linking agent.

4. The method for preparing composite particles according to any of claims 1 to 3, **characterized in that** it applies a cross-linking agent based on styrene derivatives.

5. The method for preparing the composite particles according to claim 4, **characterized in that** the cross-linking agent is divinylbenzene.

6. The method for preparing composite particles according to any of the preceding claims, **characterized in that** it applies a single cross-linking agent.

7. The method for preparing composite particles according to any of the preceding claims, **characterized in that** it applies from 0 to 35% of a styrene hydrophobic monomer and at most 5% of a fluorescent hydrophobic monomer.

8. The method for preparing composite particles according to any of claims 1 to 5, **characterized in that** it applies at most 5% of a fluorescent cross-linking agent or of a cross-linking agent based on acrylamide.

9. The method for preparing composite particles according to any of the preceding claims, **characterized in that** the organic phase comprises an alkane.

10. The method for preparing composite particles according to claim 9, **characterized in that** the alkane is octane.

11. The method for preparing composite particles according to any of the preceding claims, **characterized in that** the inorganic nanoparticles are selected from metal oxides of iron, titanium, cobalt, zinc, copper, manganese, nickel; magnetite; hematite; ferrites such as manganese, nickel, manganese-zinc ferrites; cobalt, nickel alloys; zeolites; talcum; clays such as bentonite and kaolin; alumina; silica; graphite; fluorescent crystals; colloidal gold; and carbon black.

12. The method for preparing composite particles with encapsulation by emulsion polymerization according to any of the preceding claims, **characterized in that** it comprises the steps of:
a) putting the emulsion in the presence of a surfactant;
b) adding hydrophobic cross-linking agent(s) as a monomer and
c) proceeding with polymerization.

13. The method for preparing composite particles according to claim 12, **characterized in that** the polymerization is applied in the presence of an initiator.

14. The method for preparing composite particles according to claim 13, **characterized in that** the initiator is a water-soluble radical initiator.

15. The method for preparing composite particles according to claim 14, **characterized in that** the water-soluble radical initiator is potassium persulfate.

16. The method for preparing composite particles according to any of claims 12 to 15, **characterized in that** the surfactant is an amphiphilic polymer or sodium dodecylsulfate.

17. Stable isodisperse composite particles in an organic solvent, having a diameter from 50 to 1,000 nm, which may be obtained by the method as defined in any of the preceding claims.

18. The composite particles according to claim 17, **characterized in that** they further have at their surface, reactive functional groups which may react with at least one ligand or one polymer.

19. The composite particles according to claim 17, **characterized in that** they further have a specific binding agent for a bindable substance.

20. The composite particles according to claim 19, **characterized in that** the specific binding agent is streptavidin.

21. The composite particles according to claim 17, **characterized in that** they further have at their surface, a functionalized hydrophilic layer.

22. The composite particles according to claim 21, **characterized in that** the functionalized hydrophilic layer consists of dextran.

23. Conjugates comprising a particle as defined in claim 18 or claim 21 and a ligand.

24. Conjugates comprising a particle as defined in claim 19 and a ligand bound to a bindable substance.

25. The use of the particles as defined in claim 17 to 22 or of conjugates as defined in claims 23 or 24 in a diagnostic test.

## Patentansprüche

1. Herstellungsverfahren von isodispersen, in einem organischen Lösungsmittel stabilen Verbundpartikeln mit einem Durchmesser von 50 bis 1000 nm, durch Kapselung durch Polymerisation abgereicherter Emulsion in organischer Phase, wobei die Emulsion aus Tröpfchen anorganischer Emulsion besteht, die eine organische Phase umfassen, und anorganischen Nanopartikeln, die in der organischen Phase verteilt sind, **dadurch gekennzeichnet, dass** die Polymerisation durch Verwendung von 60 bis 100 % mindestens eines Vernetzungsmittels und von 0 bis 40 % mindestens eines hydrophoben Monomers als Polymerisationsmonomer umgesetzt wird, wobei sich versteht, dass mindestens 95 % des oder der Vernetzungsmittel hydrophob sind.

2. Herstellungsverfahren von Verbundpartikeln nach Anspruch 1, **dadurch gekennzeichnet, dass** es mindestens 95 % mindestens eines Vernetzungsmittels umsetzt.

3. Herstellungsverfahren von Verbundpartikeln nach Anspruch 1, **dadurch gekennzeichnet, dass** es 100 % mindestens eines hydrophoben Vernetzungsmittels umsetzt.

4. Herstellungsverfahren von Verbundpartikeln nach einem der Ansprüche 1 bis 3, **dadurch gekennzeichnet, dass** es ein Vernetzungsmittel auf der Basis von Styrenderivaten umsetzt.

5. Herstellungsverfahren von Verbundpartikeln nach Anspruch 4, **dadurch gekennzeichnet, dass** das Vernetzungsmittel das Divinylbenzen ist.

6. Herstellungsverfahren von Verbundpartikeln nach einem der vorangehenden Ansprüche, **dadurch gekennzeichnet, dass** es ein einziges Vernetzungsmittel umsetzt.

7. Herstellungsverfahren von Verbundpartikeln nach einem der vorangehenden Ansprüche, **dadurch gekennzeichnet, dass** es 0 bis 35 % eines hydrophoben Styrenmonomers und höchstens 5 % eines fluoreszierenden hydrophoben Monomers umsetzt.

8. Herstellungsverfahren von Verbundpartikeln nach einem der Ansprüche 1 bis 5, **dadurch gekennzeichnet, dass** es höchstens 5 % eines fluoreszierenden Vernetzungsmittels oder eines Vernetzungsmittels auf der Basis von Acrylamid umsetzt.

9. Herstellungsverfahren von Verbundpartikeln nach einem der vorangehenden Ansprüche, **dadurch gekennzeichnet, dass** die organische Phase ein Alkan umfasst.

10. Herstellungsverfahren von Verbundpartikeln nach Anspruch 9, **dadurch gekennzeichnet, dass** das Alkan das Oktan ist.

11. Herstellungsverfahren von Verbundpartikeln nach einem der vorangehenden Ansprüche, **dadurch gekennzeichnet, dass** die anorganischen Nanopartikel aus den Eisen-, Titan-, Kobalt-, Zink-, Kupfer-, Mangan-, Nickelmetalloxiden, dem Magnetit, dem Hematit, den Ferriten wie den Mangan-Nickel-, Mangan-Zink-Ferriten, den Kobalt-, Nickellegierungen, den Zeoliten, dem Talg, den Tonen wie dem Bentonit und dem Kaolin, dem Aluminiumoxid, dem Siliziumoxid, dem Graphit, den fluoreszierenden Kristallen, dem kolloidalen Gold und dem Rußschwarz ausgewählt sind.

12. Herstellungsverfahren von Verbundpartikeln durch Kapselung durch Emulsionspolymerisation nach einem der vorangehenden Ansprüche, **dadurch gekennzeichnet, dass** es die Schritte umfasst, die darin bestehen:
(a) die Emulsion mit einem Tensid zusammenzubringen,
(b) ein oder hydrophobe Vernetzungsmittel als Monomer hinzuzufügen und
(c) die Polymerisation durchzuführen.

13. Herstellungsverfahren von Verbundpartikeln nach Anspruch 12, **dadurch gekennzeichnet, dass** die Polymerisation bei Anwesenheit eines Initiators umgesetzt wird.

14. Herstellungsverfahren von Verbundpartikeln nach Anspruch 13, **dadurch gekennzeichnet, dass** der Initiator ein wasserlöslicher Radikalinitiator ist.

15. Herstellungsverfahren von Verbundpartikeln nach Anspruch 14, **dadurch gekennzeichnet, dass** der wasserlösliche Radikalinitiator das Kaliumpersulfat ist.

16. Herstellungsverfahren von Verbundpartikeln nach einem der Ansprüche 12 bis 15, **dadurch gekennzeichnet, dass** das Tensidmittel ein amphiphiles Polymer oder das Natriumdodecylsulfat ist.

17. Isodisperse, in einem organischen Lösungsmittel stabile Verbundpartikel mit einem Durchmesser von 50 bis 1000 nm, die durch das Verfahren herstellbar sind, wie in einem der vorangehenden Ansprüche beschrieben.

18. Verbundpartikel nach Anspruch 17, **dadurch gekennzeichnet, dass** sie ferner auf ihrer Oberfläche reaktive Funktionsgruppen aufweisen, die imstande sind, mit mindestens einem Liganden oder einem Polymer zu reagieren.

19. Verbundpartikel nach Anspruch 17, **dadurch gekennzeichnet, dass** sie ferner ein spezielles Verbundmittel für eine bindungsfähige Substanz aufweisen.

20. Verbundpartikel nach Anspruch 19, **dadurch gekennzeichnet, dass** das spezielle Verbundmittel das Streptavidin ist.

21. Verbundpartikel nach Anspruch 17, **dadurch gekennzeichnet, dass** sie ferner auf ihrer Oberfläche eine funktionalisierte hydrophile Schicht aufweisen.

22. Verbundpartikel nach Anspruch 21, **dadurch gekennzeichnet, dass** die funktionalisierte hydrophile Schicht aus Dextran besteht.

23. Konjugate, die einen Partikel wie in Anspruch 18 oder in Anspruch 19 definiert und einen Liganden umfassen.

24. Konjugate, die einen Partikel wie in Anspruch 19 definiert und einen Liganden, der an eine bindungsfähige Substanz gebunden ist, umfassen.

25. Verwendung von Partikeln, wie in den Ansprüchen 17 bis 22 definiert, oder der Konjugate, wie in den Ansprüchen 23 oder 24 definiert, in einem diagnostischen Test.
